Europäisches Patentamt

European Patent Office

Office européen des brevets

(11) Publication number: **0 434 462 A1**

# (12) EUROPEAN PATENT APPLICATION

(21) Application number: **90314169.5**

(51) Int. Cl.[5]: **C07C 5/41, C07C 2/76**

(22) Date of filing: **21.12.90**

(30) Priority: **21.12.89 US 454531**
**29.12.89 US 459212**
**29.12.89 US 459215**

(43) Date of publication of application:
**26.06.91 Bulletin 91/26**

(84) Designated Contracting States:
**BE DE FR GB IT NL**

(71) Applicant: **MOBIL OIL CORPORATION (a New York corporation)**
**3225 Gallows Road**
**Fairfax, Virginia 22037 (US)**

(72) Inventor: **Chu, Pochen**
**1 Cranberry Place**
**Voorhees, New Jersey 08043 (US)**
Inventor: **Degnan, Thomas Francis, Jr.**
**736 Paddock Path**
**Moorestown, New Jersey 08057 (US)**
Inventor: **Johnson, Ivy Dawn**
**15 Carol Joy Road**
**Medford, New Jersey 08055 (US)**
Inventor: **Kresge, Charles Theodore**
**600 Thorncroft Drive**
**West Chester, Pennsylvania 19380 (US)**

(74) Representative: **Colmer, Stephen Gary et al**
**Mobil Court 3 Clements Inn**
**London WC2A 2EB (GB)**

(54) **Aromatization process utilizing a pillared layered silicate plus a base metal or noble metal.**

(57) Aromatization of non-aromatic hydrocarbons having 2 to 12 carbon atoms involves contacting the feed with a catalyst including gallium, zinc or a Group VIA or Group VIIIA metal incorporated into or onto a pillared layered silicate.

EP 0 434 462 A1

# AROMATIZATION OF C2-C12 HYDROCARBONS

The present invention relates to aromatization of non-aromatic $C_2$ to $C_{12}$ hydrocarbons.

The production of aromatic hydrocarbons from non-aromatic hydrocarbons using shape-selective catalyst materials is well-known. U.S. Patent No. 3,756,942 to Cattanach teaches such a method using a zinc-exchanged ZSM-5. U.S. Patent No. 4,180,689 to Davies et al teaches conversion of $C_3$-$C_{12}$ hydrocarbons to aromatics using a gallium-activated zeolite such as ZSM-5, ZSM-11, ZSM-12 or ZSM-35. Although the incorporation of a metal of mild dehydrogenation function such as zinc or gallium activates the catalyst for aromatization reactions, loss of the metal, for example by elution, commonly occurs under the high temperature reducing conditions encountered in aromatization. U.S. Patent No. 4,490,569 to Chu et al teaches a method to reduce such elution by incorporating gallium as well as zinc into a zeolite aromatization catalyst.

According to the invention, there is provided a process for producing aromatic hydrocarbons which comprises contacting a feed containing non-aromatic $C_2$ to $C_{12}$ hydrocarbons with a catalyst at a pressure of 100 to 7000 kPa (atmospheric to 1000 psig), a weight hourly space velocity of 0.05 to 300 and a temperature of 200 to 675°C, wherein said catalyst comprises a layered silicate and oxide pillars separating the layers of the silicate, said catalyst comprising at least one element selected from Groups VIA and VIIIA of the Periodic Table, gallium and zinc.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other. However, the interactions between the planes are weaker than the chemical bonds holding an individual plane together. The weaker bonds generally arise from interlayer attractions such as Van der Waals forces, electrostatic interactions, and hydrogen bonding. In those situations where the layered structure has electronically neutral sheets interacting with each other solely through Van de Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction mediated by ions located between the layers. In addition, hydrogen bonding interactions can occur directly between complementary sites on adjacent layers, or can be mediated by interlamellar bridging molecules.

Laminated materials such as clays may be modified to increase their surface area. In particular, the distance between the interlamellar layers can be increased substantially by absorption of various swelling agents, such as water, ethylene glycol, amines and ketones, which enter the interlamellar space and push the layers apart. However, the interlamellar spaces of such layered materials tend to collapse when the molecules occupying the space are removed by, for example, exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing". These values indicate the distance between, for example, the uppermost margin of one layer with the uppermost margin of its adjoining layer. If the layer thickness is known, the interlayer spacing can be determined by subtracting the layer thickness from the basal spacing.

The aromatization catalyst employed in the present invention comprises a layered silicate and oxide pillars separating the silicate layers. The oxide pillars preferably comprise at least one element selected from the group consisting of Al, B, Cr, Ga, In, Mo, Nb, Ni, Ti, Tl, W and Zr and most preferably comprise silica. The oxide pillars preferably comprise a polymeric oxide, e.g., polymeric silica or silica-alumina. The extent of polymerization of the oxide pillars is believed to affect the ultimate interlayer separation of the layered product ; that is to say, the greater the extent of polymerization occurring, the greater the interlayer distance resulting in the pillared layered silicate. The interlayer distance of the silicate may be such that polycyclic hydrocarbons can pass between adjacent layers of the silicate, preferably a distance greater than 10 angstroms or even 15 angstroms, typically 15 to 20 angstroms.

Pillared silicates containing from 5 to 50 wt% silica-alumina incorporated as the pillar material are desirable for use in the aromatization catalyst of the invention.. Particularly preferred are silicates containing from about 10 to 20 wt% silica-alumina as the pillar material.

Layered silicates suitable for producing the catalyst employed in the process of the invention include high silica alkali silicates such as magadiite, natrosilite, kenyaite, makatite, nekoite, kanemite, okenite, dehayelite, macdonaldite and rhodesite. These layered silicate materials possess a framework composed essentially of only tetrahedral sheets, i.e., silicon is coordinated with four oxygen atoms, condensed on each other. These materials lack octahedral sheets, such as those found in clays, wherein an element such as aluminum is coordinated with six oxygen atoms. High silica alkali silicates can be prepared hydrothermally from an aqueous reaction mixture containing silica and caustic at relatively moderate temperatures and pressures. These layered silicates may contain tetracoordinate framework atoms other than Si. Such layered silicates can be prepared by co-crystallizing in the presence of non-silicon tetravalent elements, e.g., those selected from the group consisting of B, Al, Ga, In and Tl as well as any other such elements which are catalytically useful when incorporated in the silicate structure. Alternatively, non-silicon framework elements already in a layered silicate may be substituted by a tetracoordinate element.

For example, synthetic magadiite materials are readily synthesized hydrothermally from a reaction mixture having the following composition in terms of molar ratios :

$SiO_2/X_2O_3$ = 10 to infinity where X can be B, Al, B, Ga, In and/or Tl or other catalytically useful metal
$M^+OH^-/SiO_2$ = 0 to 0.6, (preferably 0.1-0.6) M = an alkali metal
$H_2O/SiO_2$ = 8 - 500
$R/SiO_2$ = 0 - 0.4

where R is an organic directing agent, such as benzyltriethylammonium chloride,
benzyltrimethylammonium chloride,
dibenzyldimethylammonium chloride,
N,N'-dimethylpiperazine and triethylamine. The reaction mixture is maintained at a temperature of 100 to 200°C for anywhere from about 1 to 150 days in order to form a product having the following composition :

% N = 0 to 3, e.g., 0 to 0.3
$SiO_2/X_2O_3$ = 10 to infinity where X may be in tetrahedral or octahedral position
$M_2O/SiO_2$ = 0 to 0.5, e.g., 0.05 - 0.1

Kenyaite, a layered silica acid which is known to exist in nature as a sodium salt $Na_2Si_{22}O_{45}.H_2O$ can be prepared in the potassium form $K_2Si_{22}O_{45}.10H_2O$ in the laboratory. Synthetic kenyaite is readily synthesized hydrothermally from a reaction mixture containing inexpensive sources of silica and caustic, preferably KOH.

A layered material suitable for use in the present aromatization process can be prepared according to the method set out in U.S. Patent No. 4,859,648. In this method, the layered silicate starting material contains ion exchange sites having interspathic cations associated therewith. Such interspathic cations may include hydrogen ion, hydronium ion or alkali metal cation. The starting material is treated with a "propping" agent comprising a source of organic cation in order to effect an exchange of, or addition to, the interspathic cations resulting in the layers of the starting material being propped apart. In particular, alkylammonium cations have been found useful. Thus $C_3$ and larger alkylammonium cations, e.g., n-octylammonium, can be readily incorporated within the interlayer species of the layered silicates, serving to prop open the layers in such a way as to allow incorporation of a polymeric oxide precursor material. The extent of the interlayer spacing can be controlled by the size of the organoammonium ion employed so that use of the n-propylammonium cation can achieve a d-spacing of about 2 to 5A or an opening of about 2-3A, whereas to achieve a d-spacing opening of 10 to 20A, an n-octylammonium cation or a cation of equivalent length is required. The organic ammonium cations separating the silicate layers may also be formed in situ by reaction of the neutral amine species with interlayer hydrogen or hydronium cations of the layered silicate starting material.

The polymeric oxide pillars are formed from a precursor material which is preferably introduced between the layers of the organic "propped" species as a cationic, or more preferably, electrically neutral, hydrolyzable compound of the desired elements. The precursor material is preferably an organic compound containing said desired elements which is a liquid under ambient conditions. In particular, hydrolyzable compounds, e.g., alkoxides, of the desired elements of the pillars are utilized as the precursors. Suitable polymeric silica precursor materials include tetraalkylsilicates, e.g., tetrapropylorthosilicate, tetramethylorthosilicate and, most preferably, tetraethylorthosilicate. Introduction of interspathic polymeric oxide of an element selected from the group consisting of Al, B, Cr, Ga, In, Mo, Nb, Ni, Ti, Tl, W, and Zr to the pillar system can be achieved by contacting a hydrolyzable compound of the desired element with the organic "propped" species before, after or simultaneously with the contacting of the layered chalcogenide with the silicon compound. The hydrolyzable aluminum compound employed may be an aluminum alkoxide, e.g., aluminum isopropoxide.

The polymeric oxide precursor-containing product can be exposed to suitable conversion conditions, such as hydrolysis and/or calcination to form the layered material employed in the present invention. The hydrolysis step may be carried out by any method. Because of the effect of interspathic water on hydrolysis, the extent of hydrolysis may be modified by varying the extent to which the organic-"propped" species is dried prior to addition of the polymeric oxide presursor. The product after conversion to the polymeric oxide form may be exposed to conditions which remove organic compounds such as the organic cation propping agents, e.g., exposure to elevated temperatures such as those encountered by calcining in air or nitrogen.

After hydrolysis to produce the polymeric oxide pillars and calcination to remove the organic propping agent, the final pillared product may contain residual exchangeable cations. Such residual cations in the layered material can be ion exchanged by known methods with other cationic species to provide or alter the catalytic activity of the pillared product. In particular, gallium or zinc components or components containing elements from Groups VIA and VIIIA of the Periodic Table (as published by Fisher Scientific Company as Cat. No. 5-702-10, 1978) can be introduced by ion-exchange or impregnation techniques known in the art. Representative ion exchange techniques are disclosed in a wide variety of patents including U.S. Patent Nos. 3,140,249 ; 3,140,251 ; and 3,140,253. Generally, the aromatization catalyst of the present invention can contain about 0.1 to 20 weight percent, preferably about 0.5 to 15 weight percent of gallium, zinc, Group VIA or Group VIIIA metal.

The pillared silicates can be composited with porous inorganic oxide matrix materials such as silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The relative proportions of pillared silicate component and inorganic matrix, on an anhydrous basis, may vary widely with the silicate content ranging from 1 to 99 percent by weight and more usually in the range of from 5 to 80 percent by weight of the dry composite.

The present aromatization reaction involves contacting a suitable feedstock with the present catalyst under sufficient aromatization conditions. Such conditions may include a pressure of 100 to 7000 kPa (atmospheric to 1000 psig), preferably 100 to 1500 kPa (atmospheric to 200 psig) ; a weight hourly space velocity of about 0.05 to 300, preferably about 0.2 to 10 ; and a temperature of about 200 to 675°C, preferably about 315 to 595°C.

The feedstock may include one or more $C_2$ to $C_{12}$ non-aromatic hydrocarbons, especially, $C_6$ to $C_{12}$ paraffins, most especially, n-paraffins.

## Example 1

Silica/gallium pillared kenyaite was prepared by suspending 80 grams of a silica-kenyaite which had been swollen directly with cetyldimethylethylammonium bromide and impregnated with tetraethylorthesilicate (TEOS) in 200 g of 0.5M $Ga(NO_3)_3$. The slurry was stirred for 2 hours then filtered, air dried, and calcined for 3 hours at 540°C.

The finished material had the following properties :

| | |
|---|---|
| Surface Area, m²/g | 593 |
| Ash, 1000°C, wt% | 93.3 |
| $SiO_2$, wt% | 87.2 |
| Ga, wt% | 4.2 |
| $Al_2O_3$, ppm | 720 |

## Example 2

Silica/alumina-pillared silica-kenyaite was prepared in an analogous manner to that described in Example 1 where 200 g of the silicate was suspended in 1000 g of 0.5N $Al(NO_3)_3$. The slurry was stirred for 18 hours, filtered, air dried, and then calcined in air for 6 hours at 540°C.

The finished material had the following properties :

| | |
|---|---|
| Surface Area, m²/g | 582 |
| Ash, 1000°C, wt% | 90.58 |
| $SiO_2$, wt% | 85.3 |
| $Al_2O_3$, wt% | 2.9 |
| K, ppm | 305 |

## Example 3

A sample of ZSM-5 was prepared according to the procedure of U.S. Patent No. 3,702,886. This sample was then carefully calcined in flowing (10 v/v/min) nitrogen at a heating rate of 0.5°C/min to 538°C, held at the final temperature for three hours in nitrogen after which the atmosphere was changed to flowing (10v/v/min) dry air. After the ZSM-5 catalyst was cooled to room temperature, it was ion exchanged twice at room temperature using 5 cc/g of a 1.0N $NH_4NO_3$ solution. The catalyst was rinsed thoroughly with deionized water between exchanges. After drying the ZSM-5 catalyst overnight at 120°C, the catalyst was calcined in flowing (10 v/v/min) dry air at 0.1°C/min to 538°C and held at this temperature in flowing air for three hours.

The final catalyst had the following properties :

| | |
|---|---|
| SiO$_2$, wt% | 94.2 |
| Al$_2$O$_3$, wt% | 2.03 |
| Na, ppm | 350 |
| Ash at 1000°C, wt% | 96.5 |

Example 4

The three catalysts from Examples 1, 2, 3 were pelleted, crushed, and sized to 40/80 mesh and then loaded into 0.64 cm ID tubular quartz reactors. These catalysts were then preheated to 538°C in flowing helium (200 cc/min) at a heating rate of 30°C/min. After the catalysts reached reaction temperature (538°C), the helium stream from each reactor was diverted through a liquid sparger unit which was filled with n-hexane (98.9% n-hexane percolated through Al$_2$O$_3$). The helium-hexane mixture was cooled to 15.9°C using a circulating glycol cooled condenser to guarantee that the hexane partial pressure was 100 torr (13.3 kPa).

The helium flow rate was adjusted to obtain an n-hexane conversion ranging from 5 to 25 wt%. Over the course of one hour, six analyses were performed on each catalyst to determine activity and relative aging rates. The results of the analyses of each of these three catalysts are given in Tables 1, 2, and 3. Here, benzene selectivity is defined as follows :

Benzene Selectivity =

$$\frac{100 \ x \ wt\% \ Benzene \ in \ product}{wt\% \ non \ n\text{-}C_6 \ components \ in \ product}$$

and k is the reaction rate constant in sec$^{-1}$, assuming first order kinetics for the conversion of n-hexane.

k

EP 0 434 462 A1

## TABLE 1

### N-Hexane Conversion over Pillared Layered Gallosilicate
### Catalyst of Example 1

| Time-on Stream | Temp. (°C) | Flow RAte (cc/min) | % Conv on n-$C_6$ | Benzene Select. | k, sec$^{-1}$ x $10^4$ |
|---|---|---|---|---|---|
| 12 min | 538.5 | 22.8 | 9.9 | 30.1 | 2.07 |
| 24 min | 538.2 | 22.8 | 12.0 | 37.2 | 2.57 |
| 35 min | 538.2 | 22.8 | 11.6 | 39.5 | 2.48 |
| 47 min | 538.2 | 22.8 | 10.6 | 40.7 | 2.25 |
| 58 min | 538.2 | 22.7 | 9.9 | 41.1 | 1.94 |
| 70 min | 538.0 | 22.8 | 9.2 | 41.5 | 1.94 |

notes:  Flow Rate of Helium is measured at 25°C and 1 atm.

## TABLE 2

### N-Hexane Conversion over Pillared Layered Gallium-Free Silicate Catalyst of Example 2

Total Amount of Catalyst Loaded = 0.243 grams

| Time-on Stream | Temp. (°C) | Flow Rate (cc/min) | % Conv on n-$C_6$ | Benzene Select. | k, sec$^{-1}$ x $10^4$ |
|---|---|---|---|---|---|
| 12 min | 538.9 | 22.7 | 7.5 | 1.1 | 1.30 |
| 24 min | 539.2 | 22.7 | 6.0 | 0.7 | 1.02 |
| 36 min | 539.4 | 22.7 | 6.1 | 0.5 | 1.05 |
| 47 min | 539.2 | 22.7 | 6.4 | 0.7 | 1.08 |
| 59 min | 539.2 | 22.7 | 6.3 | 0.5 | 1.08 |
| 68 min | 539.2 | 22.7 | 6.4 | 0.5 | 1.11 |

notes: Flow Rate of Helium is measured at 25°C and 1 atm.

## TABLE 3

### N-Hexane Conversion over ZSM-5
### Catalyst of Example 3

Total Amount of Catalyst Loaded = 0.106 grams

| Time-on Stream | Temp. (°C) | Flow Rate (cc/min) | % Conv on n-$C_6$ | Benzene Select. | k, $sec^{-1}$ x $10^4$ |
|---|---|---|---|---|---|
| 11 min | 536.5 | 94.2 | 20.4 | 0.3 | 39.7 |
| 21 min | 536.2 | 93.9 | 20.4 | 0.5 | 39.7 |
| 30 min | 536.2 | 93.5 | 20.3 | 0.5 | 39.2 |
| 39 min | 536.2 | 93.2 | 20.5 | 0.5 | 39.5 |
| 49 min | 536.2 | 92.8 | 20.3 | 0.5 | 38.9 |
| 59 min | 536.0 | 92.5 | 20.3 | 0.5 | 38.7 |

notes: Flow Rate of Helium is measured at 25°C and 1 atm.

EP 0 434 462 A1

A comparison of these results shows that the benzene selectivity is the highest over the pillared gallosilicate. Note that after nearly one hour on stream the benzene selectivity of this material is still increasing. Thus, the maximum benzene selectivity for the pillared gallosilicate will be in excess of 41% compared to 0.5 to 1.0% over the pillared gallium-free layered silicate of Example 2 and approximately 0.5% for the ZSM-5 catalyst.

Table 1 and 2 also show that the gallosilicate is more active and at least as stable as the analogous gallium-free material. After an hour on stream, the rate constant of the gallosilicate has declined less than 8% (from $2.07 \times 10^{-4}$ sec $^{-1}$ to $1.94 \times 10^{-4}$ sec $^{-1}$) compared to an approximate 15% decline in activity (k drops from $1.30 \times 10^{-4}$ sec $^{-1}$ to $1.11 \times 10^{-4}$ sec $^{-1}$) for the pillared gallium-free layered silicate. This high degree of stability of the pillared layered gallosilicate is unexpected in view of its high aromatic selectivity. Generally, under equivalent reaction conditions high aromatic selectivity corresponds to faster catalyst aging rates since aromatics, and particularly alkyl aromatics, tend to condense and form coke on the catalysts, C.H. Bartholomew, "Catalyst Deactivation", Chem. Engineering, 91, November 12, 1984, p. 96. This coke in turn restricts the pores of the catalyst and covers the active sites thereby damaging conversion activity.

## Example 5

Tables 4 and 5 compare the product distributions from the conversion of n-hexane over the pillared layered gallosilicate and gallium-free silicate, respectively. These data show that the gallosilicate also consistently produces higher levels of highly desirable toluene and lower levels of less desirable $C_3$'s. The light gas selectivities $(C_1 + C_2, C_3^-, \text{ and } C_4^-)$ all decline while the toluene and benzene selectivities increase with time on stream. Again, this result is unexpected since ZSM-5 based catalysts for this type of reaction show a consistent loss in activity with time on stream.

EP 0 434 462 A1

## TABLE 4

### Product Distribution from N-Hexane Conversion over Pillared Layered Gallosilicate of Example 1

| | | | | | | |
|---|---|---|---|---|---|---|
| Time-on-Stream, min | 12 | 24 | 35 | 47 | 58 | 70 |
| Temperature, °C | 538.5 | 538.2 | 538.2 | 538.2 | 538.2 | 538.0 |
| GHSV, $hr^{-1}$ | 3734 | 3745 | 3742 | 3738 | 3734 | 3731 |
| % N-Hexane Conversion, wt% | 9.9 | 12.0 | 11.6 | 10.6 | 9.9 | 9.2 |
| **Product Distribution, wt%** | | | | | | |
| $C_1 + C_2$ | 1.56 | 1.69 | 1.47 | 1.29 | 1.17 | 1.07 |
| $C_3$'s | 1.95 | 2.07 | 1.78 | 1.58 | 1.43 | 1.32 |
| $i\text{-}C_4$ | 2.13 | 2.24 | 1.95 | 1.17 | 1.55 | 1.42 |
| $n\text{-}C_4$ | 0.36 | 0.41 | 0.01 | 0.01 | 0.01 | 0.01 |
| $C_4^=$'s | 0.01 | 0.01 | 0.01 | 0.01 | 0.01 | 0.01 |
| $i\text{-}C_5$ | 0.02 | 0.02 | 0.02 | 0.01 | 0.01 | 0.01 |
| $n\text{-}C_5$ | 0.03 | 0.03 | 0.03 | 0.03 | 0.03 | 0.03 |
| $i\text{-}C_6$ | 0.10 | 0.07 | 0.08 | 0.07 | 0.07 | 0.07 |
| $n\text{-}C_6$ | 88.96 | 86.86 | 87.30 | 88.25 | 88.98 | 89.70 |
| $C_6^=$'s | 0.41 | 0.40 | 0.45 | 0.46 | 0.48 | 0.50 |
| Benzene | 3.02 | 4.51 | 4.62 | 4.38 | 4.12 | 3.86 |
| Toluene | 0.15 | 0.31 | 0.40 | 0.42 | 0.40 | 0.37 |
| Other $C_7^+$ | 1.09 | 1.10 | 1.05 | 1.15 | 1.17 | 0.17 |
| Unknowns | 0.24 | 0.29 | 0.33 | 0.32 | 0.31 | 0.26 |
| Benzene Selectivity, wt% | 30.1 | 37.2 | 39.5 | 40.7 | 41.1 | 41.5 |
| Toluene Selectivity, wt% | 1.5 | 2.6 | 3.4 | 4.0 | 4.0 | 4.0 |
| $C_2$- Selectivity, wt% | 15.8 | 14.1 | 12.7 | 12.2 | 11.8 | 11.6 |
| $C_3$- Selectivity, wt% | 35.5 | 31.3 | 28.0 | 27.1 | 26.3 | 25.0 |
| $C_4$- Selectivity, wt% | 60.7 | 53.5 | 48.0 | 46.2 | 44.8 | 44.2 |

## TABLE 5

### Product Distribution from N-Hexane Conversion over Silica-Alumina Pillared Layered Silicate of Example 2

| | | | | | | |
|---|---|---|---|---|---|---|
| Time-on-Stream, min | 12 | 24 | 36 | 47 | 59 | 68 |
| Temperature, °C | 538.9 | 539.2 | 539.4 | 539.2 | 539.2 | 539.2 |
| GHSV, hr$^{-1}$ | 3069 | 3064 | 3064 | 3065 | 3065 | 3065 |
| % N-Hexane Conversion, wt% | 7.5 | 6.0 | 6.1 | 6.4 | 6.3 | 6.4 |
| **Product Distribution, wt%** | | | | | | |
| $C_1 + C_2$ | 1.03 | 0.88 | 0.89 | 0.89 | 0.91 | 0.92 |
| $C_3$'s | 4.73 | 3.79 | 3.85 | 3.93 | 3.98 | 4.04 |
| $i\text{-}C_4$ | 1.07 | 0.89 | 0.90 | 0.91 | 0.91 | 0.93 |
| $n\text{-}C_4$ | 0.18 | 0.15 | 0.15 | 0.15 | 0.16 | 0.15 |
| $C_4^=$'s | 0.01 | 0.01 | 0.01 | 0.01 | 0.01 | 0.01 |
| $i\text{-}C_5$ | 0.20 | 0.17 | 0.18 | 0.18 | 0.19 | 0.19 |
| $n\text{-}C_5$ | 0.06 | 0.05 | 0.05 | 0.05 | 0.05 | 0.05 |
| $i\text{-}C_6$ | 0.31 | 0.22 | 0.25 | 0.29 | 0.30 | 0.32 |
| $n\text{-}C_6$ | 91.30 | 92.90 | 92.80 | 92.51 | 92.54 | 92.45 |
| $C_6^=$'s | 0.58 | 0.58 | 0.59 | 0.61 | 0.60 | 0.61 |
| Benzene | 0.08 | 0.04 | 0.03 | 0.04 | 0.03 | 0.03 |
| Toluene | 0.02 | 0.02 | 0.01 | 0.01 | 0.01 | 0.01 |
| Other $C_7^+$ | 0.25 | 0.33 | 0.17 | 0.28 | 0.17 | 0.18 |
| Unknowns | 0.40 | 0.13 | 0.29 | 0.13 | 0.13 | 0.13 |
| Benzene Selectivity, wt% | 1.07 | 0.69 | 0.50 | 0.65 | 0.45 | 0.46 |
| Toluene Selectivity, wt% | 0.2 | 0.3 | 0.2 | 0.1 | 0.1 | 0.1 |
| $C_2^-$ Selectivity, wt% | 13.7 | 14.7 | 14.6 | 13.9 | 14.4 | 14.4 |
| $C_3^-$ Selectivity, wt% | 76.8 | 77.8 | 77.7 | 75.3 | 77.6 | 77.5 |
| $C_4^-$ Selectivity, wt% | 93.6 | 95.3 | 95.1 | 92.0 | 94.8 | 94.5 |

EP 0 434 462 A1

## Example 6

A tetraethylorthosilicate-impregnated kenyaite (10 g, 60% ash) was added to an aqueous $Cr(NO_3)_3$ solution (120 g, 0.5M). The slurry was stirred for 2 hours, filtered, air-dried, and calcined at 540°C for 6 hours in air. The composition and surface area are listed in Table 6.

## Example 7

A silica-pillared (Si) kenyaite (2 g) was impregnated with an aqueous $Cr(NO_3)_3$ solution (0.223 g in 2 g $H_2O$). The material was calcined at 540°C for 3 hours in air. The composition and surface area are listed in Table 6.

## Example 8

A silica-pillared (Si) kenyaite (5 g) was impregnated with an aqueous $Cr(NO_3)_3$ solution (1.2 g in 5 g $H_2O$). The material was rolled for 1 hour, then calcined at 540°C for 3 hours in air. The composition and surface area are listed in Table 6.

## Example 9

An amorphous silica gel (Ultrasil, 5 g) was impregnated with an aqueous $Cr(NO_3)_3$ solution (1.2 g in 7 g $H_2O$). The material was rolled for 2 hours, then calcined at 540°C for 3 hours. The composition and surface area are listed in Table 6.

## Example 10

An amorphous silica gel (Hi-Sil, 5 g) was treated the same as described in Example 9. The composition and surface area are listed in Table 6.

## Example 11

Kaiser alumina (15 g) was impregnated with an aqueous $Cr(NO_3)_3$ solution (3.6 g in 15 g $H_2O$). The material was rolled for 1 hour, then calcined at 540°C for 3 hours. The composition and surface area are listed in Table 6.

## Example 12

A sample similar to that described in Example 11 was prepared. Only the alumina was calcined at 540°C for 3 hours prior to the treatment. The composition and surface area are listed in Table 6.

## Example 13

Catalyst samples prepared in the above Examples were tested for dehydrogenation of $C_6$ and $C_7$ alkanes. Fixed weights of the catalysts were charged into a small Pyrex reactor. Normal hexane or other specified feed such as n-heptane or cyclohexane was charged over the catalyst bed at 2 WHSV. The operating conditions were set at 480 to 540°C (900 to 1000°F) under atmospheric pressure. After being on stream for a specified time, a product sample was analyzed by online gas chromatography using a fused silica capillary column. The results are listed in Tables 7 and 8, from which it will be seen that the catalysts of the invention exhibit improved conversion and selectivity as compared with amorphous silica catalysts and less unfavorable, non-selective, side reactions, such as cracking, as compared with alumina catalysts.

## Example 14

Propane was dehydrogenated over the catalyst of Example 6. Process conditions and results are summarized in Table 9.

## TABLE 6

### Composition and Surface Area of Cr-Silicates

| Example | 6 | 7 | 8 | 9 | 10 | 11 | 12 |
|---|---|---|---|---|---|---|---|
| **Property** | | | | | | | |
| **Composition wt%:** | | | | | | | |
| $SiO_2$,% | 89.7 | 81.9 | 85.3 | 88.5 | 86.7 | 0.44 | 0.26 |
| Cr,% | 2.9 | 1.1 | 2.1 | 2.6 | 2.9 | 1.2 | 1.7 |
| $Al_2O_3$ | 805ppm | 890ppm | 895ppm | 1050ppm | 0.35% | 91.8% | 90.3% |
| K | <2.4ppm | 0.02% | 0.03% | <0.01% | 0.02% | <0.01% | <0.01% |
| Ash 1000°C,% | 91.87 | 83.94 | 86.85 | 92.83 | 96.40 | 84.38 | 90.62 |
| Surface Area ($m^2$/g) | 679 | 658* | 658* | 159 | 114 | 288 | 260 |

\* Surface area of silica-pillared kenyaite prior to impregnation.

EP 0 434 462 A1

EP 0 434 462 A1

## TABLE 7

### Dehydrocyclization of Alkanes over
### Cr-Containing SiO2-Pillared Silicates

| Catalyst | Example 6 | | | | Example 7 | | Example 8 |
|---|---|---|---|---|---|---|---|
| **Test Conditions:** | | | | | | | |
| Temp (°F) | 1000 | 900 | 1000 | 1000 | 1000 | 1000 | 1000 |
| (°C) | 540 | 480 | 540 | 540 | 540 | 540 | 540 |
| Feed | Hexane | Hexane | Hexane | Heptane | $CyC_6$ | Hexane | Hexane |
| WHSV | 2.8 | 2 | 2 | 2 | 2 | 1.6 | 1.4 |
| TOS (min) | 5 | 10 | 10 | 10 | 10 | 5 | 5 |
| Conversion | 21.1 | 7.7 | 14.4 | 13.6 | 14.1 | 16.9 | 21.6 |
| **Selectivity** | | | | | | | |
| to Aromatics | 40.5 | 20.8 | 33.3 | 58.1 | 83.0 | 42.6 | 51.9 |
| **Product Distribution:** | | | | | | | |
| Light Products (%) (excluding feed) | 7.2 | 6.1 | 9.6 | 5.7 | 2.3 | 9.7 | 10.4 |
| Benzene | 4.7 | 1.6 | 4.7 | N.D | 11.7 | 6.9 | 10.4 |
| Toluene | 0.2 | N.D | 0.1 | 7.2 | N.D | 0.3 | 0.1 |
| $C_7$ Aromatics | N.D | N.D | N.D | 0.7 | N.D | N.D | 0.7 |

N.D = Not Detected

## TABLE 8

### Dehydrocyclization of Alkanes over Cr-Containing Silicas and Aluminas

| | Example 9 | | Example 10 | | Example 11 | | Example 12 | |
|---|---|---|---|---|---|---|---|---|
| **Test Conditions:** | | | | | | | | |
| Temp (°F) | 900 | 1000 | 900 | 1000 | 900 | 1000 | 900 | 1000 |
| (°C) | 480 | 540 | 480 | 540 | 480 | 540 | 480 | 540 |
| Feed | Hexane | Hexane | Hexane | Hexane | Hexane | Hexane | Hexane | Hexane |
| WHSV | 2 | 2 | 2 | 2 | 2 | 2 | 2 | 2 |
| TOS (min) | 10 | 10 | 10 | 10 | 10 | 10 | 10 | 10 |
| Conversion | 3.0 | 7.9 | 3.3 | 7.6 | 15.18 | 38.08 | 23.57 | 45.62 |
| Selectivity | | | | | | | | |
| to Aromatics | 16.7 | 17.7 | 15.1 | 22.4 | 22.8 | 41.9 | 32.9 | 52.0 |
| **Product Distribution:** | | | | | | | | |
| Light Products (%) (excluding feed) | 2.6 | 6.6 | 2.7 | 6.0 | 11.74 | 22.14 | 15.81 | 21.89 |
| Benzene | 0.2 | 0.7 | 0.3 | 1.2 | 3.44 | 15.81 | 7.76 | 23.62 |
| Toluene | 0.3 | 0.7 | 0.2 | 0.5 | 0.02 | 0.13 | 0.0 | 0.11 |
| $C_7$ Aromatics | N.D | N.D | N.D | 0.7 | N.D | N.D | N.D | 0.11 |

N.D = Not Detected

EP 0 434 462 A1

## TABLE 9

### Dehydrogenation of Propane

Test Conditions:

| | | |
|---|---|---|
| Temp °F (°C) | 1000 (540) | 1100 (590) |
| Feed | -------Propane---------- | |
| WHSV | 2 | 2 |
| TOS (min) | 10 | 10 |

| | | |
|---|---|---|
| Conversion | 18.7 | 38.4 |
| Selectivity to $C_3$ Olefin | 74.2 | 62.1 |

Product Distribution:

| | | |
|---|---|---|
| $C_2^-$ | 4.7 | 14.0 |
| $C_3^=$ | 13.9 | 23.8 |
| $C_4^+$ | 0.1 | 0.5 |

## Claims

1. A process for producing aromatic hydrocarbons which comprises contacting a feed containing non-aromatic $C_2$ to $C_{12}$ hydrocarbons with a catalyst at a pressure of 100 to 7000 kPa (atmospheric to 1000 psig), a weight hourly space velocity of 0.05 to 300 and a temperature of 200 to 675°C, wherein said catalyst comprises a layered silicate and oxide pillars separating the layers of the silicate, said catalyst comprising at least one element selected from Groups VIA and VIIIA of the Periodic Table, gallium and zinc.

2. The process of claim 1, wherein said pressure is 100 to 1500 kPa (atmospheric to 200 psig), said weight hourly space velocity is 0.2 to 20 and said temperature is 315 to 595°C.

3. The process of claim 1 or claim 2, wherein the pillars comprise polymeric silica.

4. The process of any preceding claim, wherein said layered silicate is magadiite.

5. The process of any one of claims 1 to 3, wherein said layered silicate is kenyaite.

6. The process of any preceding claim, wherein said at least one element is gallium.

7. The process of any one of claims 1 to 5, wherein said at least one element is chromium.

European Patent Office

**EUROPEAN SEARCH REPORT**

Application Number

| | DOCUMENTS CONSIDERED TO BE RELEVANT | | EP 90314169.5 |
|---|---|---|---|

| Category | Citation of document with indication, where appropriate, of relevant passages | Relevant to claim | CLASSIFICATION OF THE APPLICATION (Int. Cl.5) |
|---|---|---|---|
| X | EP - A2 - 0 222 597 (MOBIL OIL CORPORATION) * Page 5, line 65; page 6, line 4; page 7, lines 2,10 * | 1,3 | C 07 C 5/41 C 07 C 2/76 |
| P,A | US - A - 4 935 573 (AUFDEMBRINK et al.) * Claims * | 1-3,6, 7 | |
| A | US - A - 4 861 933 (NEMET-MAVRODIN) * Claims * | 1,2,6 | |
| A | DE - A - 1 910 171 (GULF RESEARCH) * Example 1 * | 1,2,7 | |

TECHNICAL FIELDS SEARCHED (Int. Cl.5)

C 07 C 5/00
C 07 C 2/00

The present search report has been drawn up for all claims

| Place of search | Date of completion of the search | Examiner |
|---|---|---|
| VIENNA | 25-03-1991 | KÖRBER |

EPO FORM 1503 03.82 (P0401)